# EUROPEAN PATENT APPLICATION

(11) **EP 4 667 426 A1**
(43) Date of publication of application: **24.12.2025**
(21) Application number: 23922665.7
(22) Date of filing: 15.02.2023
(51) Int. Cl.: C02F 11/04, C02F 3/28

(54) **BIOGAS PRODUCTION SYSTEM**

(71) Applicant: JTEKT Corporation, Kariya-shi, Aichi 448-8652 (JP)
(72) Inventor: MITSUI Tetsuya, Kariya-shi, Aichi 448-8652 (JP); HASEGAWA Sho, Kariya-shi, Aichi 448-8652 (JP); IMOTO Yoshinori, Kariya-shi, Aichi 448-8652 (JP)
(74) Representative: TBK
(86) International application number: PCT/JP2023/005150
(87) International publication number: WO 2024/171328

(57) **Abstract**

A biogas production system (1) is configured to produce biogas (G) using, as raw materials, waste water-soluble coolants (C) collected from machining devices that perform cutting or grinding. The biogas production system (1) includes: a plurality of storage tanks (2) configured to separate by type and store the waste water-soluble coolants (C) collected from the machining devices; concentration measuring units (21) configured to measure concentrations of the waste water-soluble coolants (C) in the storage tanks (2); a mixing device (3) configured to prepare a coolant mixture (M) containing a plurality of the waste water-soluble coolants (C) by mixing the waste water-soluble coolants (C) in the storage tanks (2) at a ratio calculated based on the concentrations of the waste water-soluble coolants; and a fermentation device (4) configured to produce the biogas (G) by fermenting the coolant mixture (M) supplied from the mixing device (3) using microorganisms.

## Description

### TECHNICAL FIELD

The present disclosure relates to a biogas production system.

### BACKGROUND ART

In recent years, the enormousness of the effect of carbon dioxide emitted by human activities on the global environment has become a concern, and efforts to achieve carbon neutrality are desired from the viewpoint of reducing the burden on the global environment. For example, Patent Document 1 describes an organic waste treatment device that treats organic waste to produce methane.

When performing machining such as cutting, grinding, and polishing, water-soluble coolants are used for the purposes of reducing friction between a workpiece and a tool, and for cooling and cleaning the workpiece. Hitherto, a waste water-soluble coolant that is used in machining and discharged from a machining device has been discarded after being subjected to detoxifying treatment. If organic matter such as mineral oil contained in the waste water-soluble coolant could be recycled, however, it would be useful in achieving carbon neutrality. Therefore, there is a demand to develop a technology to ferment the organic matter in the waste water-soluble coolant and turn it into a resource.

### Related Art Documents

### Patent Documents

Patent Document 1: Japanese Unexamined Patent Application Publication No. 2012-115812 (JP 2012-115812 A)

### SUMMARY OF THE INVENTION

### Problem to be Solved by the Invention

When producing biogas by fermenting organic waste, it is important to supply the organic waste in an easily fermentable state to microorganisms in order to produce biogas efficiently. However, the components of the waste water-soluble coolant discharged from the machining device vary depending on the type of machining. Further, the amount of the waste water-soluble coolant discharged from the machining device changes daily depending on the amount of machining performed by the machining device, etc. Therefore, when fermenting the waste water-soluble coolant, there is a problem of difficulty in stably producing biogas because the microorganisms may be unable to adapt to fluctuations in the concentration and components of the waste water-soluble coolant.

The present disclosure has been made in view of such issues, and provides a biogas production system that can stably produce biogas from a waste water-soluble coolant used in machining.

### Means for Solving the Problem

One aspect of the present disclosure is a biogas production system configured to produce biogas using, as raw materials, waste water-soluble coolants collected from machining devices that perform machining. The biogas production system includes: a plurality of storage tanks configured to separate by type and store the waste water-soluble coolants collected from the machining devices; concentration measuring units configured to measure concentrations of the waste water-soluble coolants in the storage tanks; a mixing device configured to prepare a coolant mixture containing a plurality of the waste water-soluble coolants by mixing the waste water-soluble coolants in the storage tanks at a ratio calculated based on the concentrations of the waste water-soluble coolants; and a fermentation device configured to generate the biogas by fermenting the coolant mixture supplied from the mixing device using microorganisms.

### Effects of the Invention

The biogas production system is configured to separate by type the waste water-soluble coolants collected from the machining devices and store them in the plurality of storage tanks. The biogas production system is further configured to measure the concentrations of various types of waste water-soluble coolant stored in the storage tanks using the concentration measuring units, and mix the various types of waste water-soluble coolant at the ratio according to the concentrations in the mixing device. By mixing the plurality of types of waste water-soluble coolant to prepare the coolant mixture, the coolant mixture can be adjusted to a state in which it is easily fermentable by the microorganisms in the fermentation device. By fermenting the coolant mixture in the fermentation device, the biogas can be generated stably.

As described above, according to the above aspect, it is possible to provide the biogas production system that can stably produce biogas from the waste water-soluble coolant used in machining.

### BRIEF DESCRIPTION OF THE DRAWINGS

[FIG. 1] FIG. 1 is an explanatory diagram showing a schematic configuration of a biogas production system according to a first embodiment.
[FIG. 2] FIG. 2 is an explanatory diagram showing a schematic configuration of a biogas production system including a dilution device according to a second embodiment.
[FIG. 3] FIG. 3 is an explanatory diagram showing a schematic configuration of a biogas production system configured to dilute a coolant mixture using a digested liquid according to a third embodiment.
[FIG. 4] FIG. 4 is an explanatory diagram showing a schematic configuration of a biogas production system including a metal removal device and an inhibitor conversion device according to a fourth embodiment.

### MODES FOR CARRYING OUT THE INVENTION

### (First Embodiment)

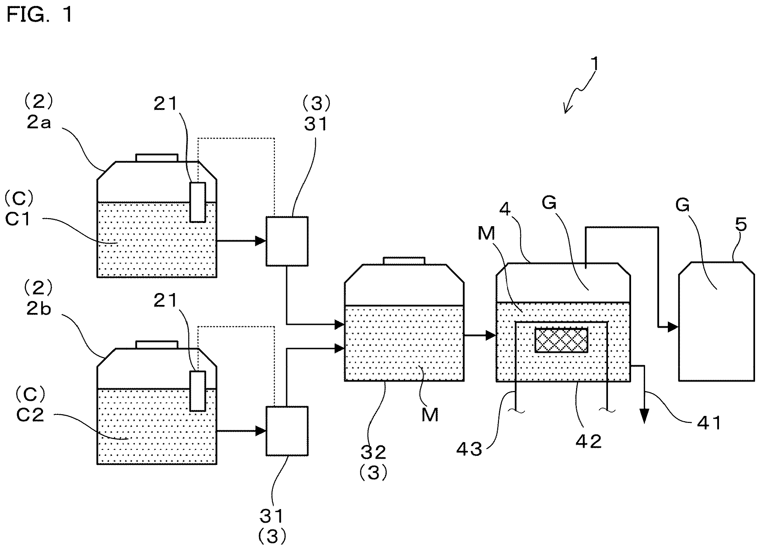

An embodiment of the biogas production system will be described with reference to FIG. 1. A biogas production system 1 of the present embodiment is configured to produce biogas G using a waste water-soluble coolant C collected from a machining device as a raw material. As shown in FIG. 1, the biogas production system 1 includes a plurality of storage tanks 2 configured to separate by type and store waste water-soluble coolants C collected from machining devices, concentration measuring units 21 configured to measure the concentrations of the waste water-soluble coolants C in the storage tanks 2, a mixing device 3 configured to prepare a coolant mixture M containing a plurality of waste water-soluble coolants C by mixing the waste water-soluble coolants C in the storage tanks 2 at a ratio calculated based on the concentrations, and a fermentation device 4 configured to produce the biogas G by fermenting the coolant mixture M using microorganisms. The configuration of each component of the biogas production system 1 will be described in detail below.

The biogas production system 1 includes the plurality of storage tanks 2 for storing the waste water-soluble coolants C collected from the machining devices. The storage tanks 2 are provided most upstream among the devices constituting the biogas production system 1, and are configured to supply the waste water-soluble coolants C to a device connected downstream of the storage tanks 2. For example, the storage tanks 2 of the present embodiment are connected to the mixing device 3, and are configured to supply the waste water-soluble coolants C to the mixing device 3.

The waste water-soluble coolants C to be used as raw materials in the biogas production system 1 are collected from various machining devices such as a cutting device, a grinding device, and a polishing device. The waste water-soluble coolant C may contain not only mineral oil and a surfactant contained in a water-soluble coolant for machining, but also organic matter resulting from alteration of components in the water-soluble coolant.

The waste water-soluble coolants C can be classified into various types depending on their compositions, characteristics, applications, etc. For example, the waste water-soluble coolants C can be classified into three types depending on their compatibilities with water: an emulsion type in which cutting oil components are dispersed in water, a solution type in which cutting oil components are dissolved in water, and a soluble type that is an intermediate type between the emulsion type and the solution type. The waste water-soluble coolants C can also be classified depending on the types of machine tools in which they were used. Further, the waste water-soluble coolants C can be classified depending on similarities of the components.

The waste water-soluble coolants C having various compositions as described above are separated by type and stored in the storage tanks 2 of the biogas production system 1. The number of storage tanks 2 provided in the biogas production system 1 may be two or more, and can be set as appropriate depending on the desired manner of separation of the waste water-soluble coolants. For example, the biogas production system 1 of the present embodiment includes two storage tanks 2 (2a, 2b), and is configured to store different types of waste water-soluble coolant C (C1, C2) in the storage tanks 2a, 2b.

The method for separating the waste water-soluble coolants C to be stored in the storage tanks 2 may be set as appropriate depending on the types of the waste water-soluble coolants C collected from the machine tools. For example, when the waste water-soluble coolants C collected from the machine tools can be separated depending on types based on the compatibilities with water as described above, the waste water-soluble coolants C can be separated depending on types based on the compatibilities with water and stored in the storage tanks 2. For example, when the waste water-soluble coolants C collected from the machine tools can be separated depending on similarities of the components, the waste water-soluble coolants C can be separated depending on types based on the similarities of the components and stored in the storage tanks 2.

Each storage tank 2 is provided with the concentration measuring unit 21 configured to measure the concentration of the waste water-soluble coolant C in the storage tank 2. The concentration measuring unit 21 can take various forms as long as it is configured to measure the concentration of organic matter in the waste water-soluble coolant C. For example, the concentration measuring unit 21 may be a concentration meter configured to measure the concentration of organic matter in the waste water-soluble coolant C based on various physical property values such as a refractive index, density, and conductivity of the waste water-soluble coolant C.

The concentration measuring unit 21 preferably includes a COD sensor configured to measure the chemical oxygen demand (i.e., COD) of the waste water-soluble coolant C. The COD of the waste water-soluble coolant C generally increases as the concentration of organic matter in the waste water-soluble coolant C increases. Therefore, the concentration of organic matter in the waste water-soluble coolant can be measured by measuring the COD of the waste water-soluble coolant C. By setting the mixing ratio of the waste water-soluble coolants C in the mixing device 3 based on the concentrations of the waste water-soluble coolants C measured in this manner, the concentration of organic matter in the coolant mixture M can be controlled more accurately. As a result, a coolant mixture M that is more easily fermentable can be prepared, and the efficiency of production of the biogas G can further be increased.

The mixing device 3 is connected downstream of the plurality of storage tanks 2. The mixing device 3 is configured to mix the waste water-soluble coolants C in the storage tanks 2 at a ratio calculated based on the concentrations, thereby preparing the coolant mixture M containing the plurality of waste water-soluble coolants C.

The mixing device 3 of the present embodiment includes a mixing tank 32 configured to mix the waste water-soluble coolants C stored in the storage tanks 2, and coolant pumps 31 that pump out the waste water-soluble coolants C from the storage tanks 2 to the mixing tank 32. The coolant pump 31 is configured to adjust the flow rate of the waste water-soluble coolant C from each storage tank 2 to the mixing tank 32 such that the concentration of the coolant mixture M reaches a preset target concentration based on the concentration of the waste water-soluble coolant C in each storage tank 2 measured by the concentration measuring unit 21. The mixing tank 32 is connected to the fermentation device 4, and is configured such that the coolant mixture M prepared in the mixing tank 32 can be supplied to the fermentation device 4.

The specific form of the mixing device 3 is not limited to the form of the present embodiment. For example, the mixing device 3 need not include the mixing tank 32. In this case, the waste water-soluble coolants C pumped out from the coolant pumps 31 may be supplied to the fermentation device 4, and the coolant mixture M may be prepared and fermented in the fermentation device 4. To adjust the mixing ratio of the waste water-soluble coolants C in the mixing device 3, various fluid devices such as a flow regulating valve may be used in addition to the coolant pumps 31.

By mixing the waste water-soluble coolants C separated in advance by type at a ratio according to the concentrations, a coolant mixture M that is easily fermentable by the microorganisms in the fermentation device 4 can be prepared in the mixing tank 32.

The target concentration of the coolant mixture M in the mixing device 3 of the present embodiment may be set as appropriate depending on the type and amount of the microorganisms to be used in the fermentation device 4. For example, when the upper and lower limits of the concentration at which the coolant mixture M can be fermented in the fermentation device 4 are known, the target concentration of the coolant mixture M may be set to a concentration between the upper and lower limits of the concentration at which the coolant mixture M can be fermented.

The fermentation device 4 is connected downstream of the mixing device 3. The fermentation device 4 is configured to generate the biogas G by causing the microorganisms to ferment the coolant mixture M prepared in the mixing device 3. The biogas G generated in the fermentation device 4 is collected in a biogas tank 5 described later. The fermentation device 4 includes a drain pipe 41 through which a digested liquid I that is the coolant mixture M after the fermentation by the microorganisms is discharged to the outside of the biogas production system 1. The drain pipe 41 is configured such that the excess digested liquid I can be discharged to the outside of the biogas production system 1 when the amount of the digested liquid I in the fermentation device 4 is larger than a predetermined threshold value.

In the fermentation device 4, the manner of contact between the coolant mixture M and the microorganisms is not particularly limited. For example, the fermentation device 4 may be configured to directly mix the coolant mixture M with the microorganisms that can ferment the coolant mixture M to bring the coolant mixture M into contact with the microorganisms. The fermentation device 4 may be configured to mix the coolant mixture M with a composition containing the microorganisms (e.g., soil or sludge) to bring the coolant mixture M into contact with the microorganisms.

As shown in FIG. 1, the fermentation device 4 may further include a microorganism carrier 42 that carries the microorganisms that can ferment the coolant mixture M, and may be configured to bring the coolant mixture M into contact with the microorganism carrier 42. As the carrier, for example, a porous body such as a resin sponge, a tubular body such as a plastic molded into a tubular shape, or a porous body with a support frame in which a plastic support frame is provided around the porous body can be used.

The fermentation device 4 may further include a fermentation promotion device for promoting the fermentation of the coolant mixture M. Examples of the fermentation promotion device include an agitation device (not shown) for further improving the efficiency of contact between the coolant mixture M and the microorganisms, a temperature adjustment device 43 (see FIG. 1) for adjusting the temperature of the coolant mixture M to increase the activity of the microorganisms, and a pH adjustment device (not shown).

When the temperature adjustment device 43 is provided as in the fermentation device 4 of the present embodiment, the temperature adjustment device may be configured to adjust the temperature of the coolant mixture M by, for example, utilizing waste heat generated in a factory. In this case, the energy required to operate the biogas production system 1 can further be reduced, and the environmental load when producing biogas can further be reduced.

The biogas G generated from the fermentation device 4 may be a mixture of a plurality of types of gas. The types and amounts of the gases contained in the biogas G vary depending on the types of substances contained in the coolant mixture M, the type of the microorganisms used in the fermentation device 4, etc. For example, the biogas G may contain a hydrocarbon gas such as methane (CH₄), carbon dioxide (CO₂), or hydrogen sulfide (H₂S). From the viewpoint of usefulness as a resource, the fermentation device 4 is preferably configured to generate biogas G containing methane. To generate such biogas G, the microorganisms to be used in the fermentation device 4 preferably include, for example, methanogens.

More preferably, the microorganisms to be used in the fermentation device 4 include methanogens and acid-producing bacteria. The coolant mixture M contains organic matter such as hydrocarbons constituting mineral oil. It is considered that, when the methanogens and the acid-producing bacteria are brought into contact with the coolant mixture M in the fermentation device 4, hydrocarbons etc. in the waste water-soluble coolant C are digested by the acid-producing bacteria to produce fatty acids, hydrogen, and carbon dioxide. It is considered that these products are further digested by the methanogens to produce biogas containing methane more efficiently.

The microorganism carrier 42 in the fermentation device 4 of the present embodiment carries the methanogens. Therefore, the fermentation device 4 is configured to generate the biogas G containing methane.

The biogas production system 1 may include the biogas tank 5 configured to store the biogas. The biogas tank 5 is provided most downstream among the devices constituting the biogas production system 1, and is configured to store the biogas G supplied from the device connected upstream of the biogas tank 5. The biogas tank 5 in the biogas production system 1 of the present embodiment is connected to the fermentation device 4, and is configured to store the biogas G generated in the fermentation device 4.

As described above, the biogas production system 1 of the present embodiment is configured to separate by type the waste water-soluble coolants C collected from the machining devices and store them in the plurality of storage tanks 2. The biogas production system 1 is further configured to measure the concentrations of various types of waste water-soluble coolant C stored in the storage tanks 2 using the concentration measuring units 21, and mix the various types of waste water-soluble coolant C at a ratio according to the concentrations in the mixing device 3. By mixing the plurality of types of waste water-soluble coolant C to prepare the coolant mixture M, the coolant mixture M can be adjusted to a state in which it is easily fermentable by the microorganisms in the fermentation device 4. By fermenting the coolant mixture M in the fermentation device 4, the biogas G can be generated stably.

As described above, according to the above aspect, it is possible to provide the biogas production system that can stably produce biogas from the waste water-soluble coolant used in machining.

### (Second Embodiment)

The present embodiment illustrates an example of a biogas production system 102 including a dilution device 6 configured to dilute the coolant mixture M. Among the symbols used in the present embodiment onward, the same symbols as those used in the previously described embodiment represent the same components as those in the previously described embodiment unless otherwise specified.

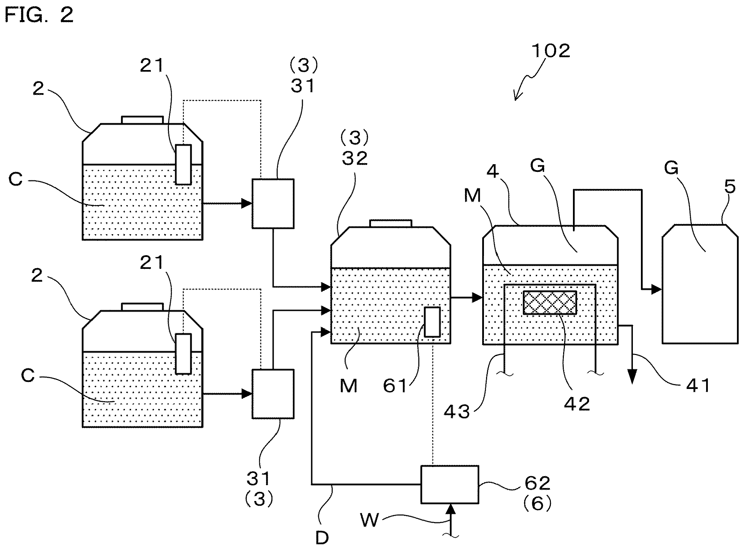

As shown in FIG. 2, the biogas production system 102 of the present embodiment includes the plurality of storage tanks 2, the concentration measuring units 21 configured to measure the concentrations of the waste water-soluble coolants C in the storage tanks 2, the mixing device 3 configured to mix the waste water-soluble coolants C in the storage tanks 2 to prepare the coolant mixture M, and the fermentation device 4 configured to ferment the coolant mixture M. The configurations of the storage tank 2, the concentration measuring unit 21, the mixing device 3, and the fermentation device 4 in the biogas production system 102 are similar to the configurations of the components in the biogas production system 1 of the first embodiment.

The biogas production system 102 further includes the dilution device 6 configured to supply, to at least one type of liquid selected from the group consisting of the waste water-soluble coolant C and the coolant mixture M, a dilution liquid D for diluting the liquid, and a second concentration measuring unit 61 configured to measure the concentration of the coolant mixture M. The dilution device 6 is configured to adjust the supply amount of the dilution liquid D based on the concentration of the coolant mixture M.

The dilution device 6 of the present embodiment includes a water pump 62 configured to pump tap water W as the dilution liquid D to the mixing tank 32. The water pump 62 is configured to supply the tap water W as the dilution liquid D into the mixing tank 32 such that the concentration of the coolant mixture M measured by the second concentration measuring unit 61 reaches a predetermined target concentration.

The specific form of the dilution device 6 is not limited to the form of the present embodiment, and various forms are possible as long as the coolant mixture M to be used for fermentation can be diluted.

Although illustration is omitted in the figure, for example, the dilution device 6 is not limited to being connected to the mixing tank 32, and the dilution device 6 can be connected to the storage tank 2 or the fermentation device 4. More specifically, the dilution device 6 may be connected to the storage tank 2 instead of the mixing tank 32, and configured to supply the dilution liquid into the storage tank 2. The dilution device 6 may be connected to both the storage tank 2 and the mixing tank 32, and configured to supply the dilution liquid D to any one of the storage tank 2 and the mixing tank 32. Further, the dilution device 6 may be connected to the fermentation device 4, and configured to supply the dilution liquid D into the fermentation device 4.

The dilution liquid D to be used in the dilution device 6 is not limited to the tap water W, and may be any liquid having a lower concentration of organic matter than the coolant mixture M to be fermented in the fermentation device 4. For example, the digested liquid that is the coolant mixture M fermented in the fermentation device 4 and reduced in the organic matter concentration can be used as the dilution liquid D to be used in the dilution device 6.

As shown in FIG. 2, the second concentration measuring unit 61 of the present embodiment is provided in the mixing tank 32 of the mixing device 3, and is configured to measure the concentration of the coolant mixture M in the mixing tank 32. The second concentration measuring unit 61 can take various forms as long as it is configured to measure the concentration of the coolant mixture M. For example, the second concentration measuring unit 61 may be a concentration meter configured to measure the concentration of the coolant mixture M based on various physical property values such as a refractive index, density, and conductivity of the coolant mixture M.

The second concentration measuring unit 61 preferably includes a COD sensor configured to measure the concentration based on the chemical oxygen demand of the coolant mixture M. In this case, the second concentration measuring unit 61 can measure the concentration of the coolant mixture M based on the COD of the coolant mixture M. The dilution device 6 can prepare a coolant mixture M that is more easily fermentable by setting the supply amount of the dilution liquid D based on the determined concentration of the coolant mixture M. As a result, the efficiency of production of the biogas G can further be increased.

By diluting the waste water-soluble coolant C and/or the coolant mixture M using the dilution liquid D, the concentration of the coolant mixture M to be supplied to the fermentation device 4 can be adjusted more easily to a concentration suitable for fermentation. Thus, the biogas G can be produced more stably.

### (Third Embodiment)

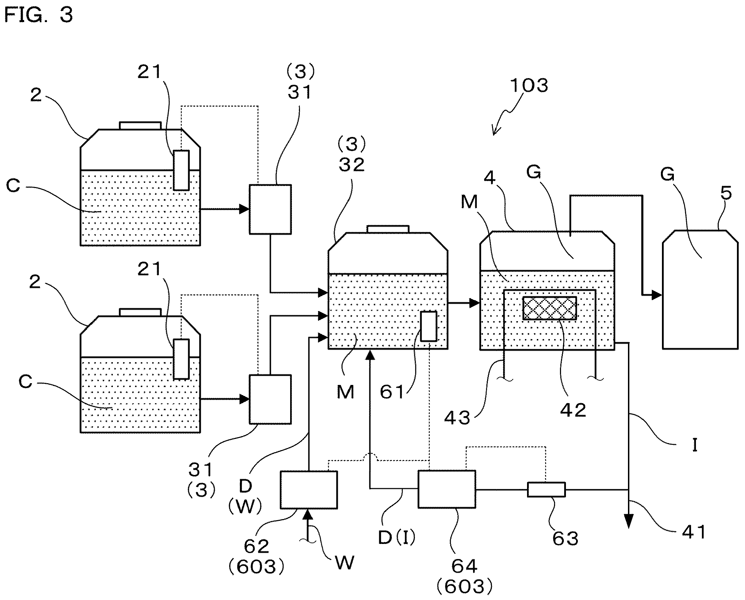

The present embodiment illustrates an example of a biogas production system 103 configured such that the digested liquid I of the coolant mixture M can be used as the dilution liquid D. As shown in FIG. 3, the biogas production system 103 of the present embodiment includes the plurality of storage tanks 2, the concentration measuring units 21 configured to measure the concentrations of the waste water-soluble coolants C in the storage tanks 2, the mixing device 3 configured to mix the waste water-soluble coolants C in the storage tanks 2 to prepare the coolant mixture M, and the fermentation device 4 configured to ferment the coolant mixture M. The configurations of the storage tank 2, the concentration measuring unit 21, the mixing device 3, and the fermentation device 4 in the biogas production system 102 are similar to those in the biogas production system 1 of the first embodiment. The mixing tank 32 of the mixing device 3 is provided with the second concentration measuring unit 61 configured to measure the concentration of the coolant mixture M. The configuration of the second concentration measuring unit 61 is similar to that in the biogas production system 102 of the second embodiment.

The biogas production system 103 of the present embodiment further includes a third concentration measuring unit 63 configured to measure the concentration of the digested liquid I made from the coolant mixture M fermented in the fermentation device 4. A dilution device 603 of the present embodiment uses the digested liquid I as the dilution liquid D, and is configured to adjust the supply amount of the dilution liquid D based on the concentration of the coolant mixture M and the concentration of the digested liquid I.

More specifically, the dilution device 603 includes the water pump 62 configured to supply the tap water W as the dilution liquid D to the mixing tank 32, and a digested liquid pump 64 configured to supply the digested liquid I as the dilution liquid to the mixing tank 32. The water pump 62 is configured to supply the tap water W as the dilution liquid D into the mixing tank 32 such that the concentration of the coolant mixture M measured by the second concentration measuring unit 61 reaches a predetermined target concentration.

The digested liquid pump 64 is connected to both the drain pipe 41 of the fermentation device 4 and the mixing tank 32, and is configured to supply at least part of the digested liquid I discharged from the fermentation device 4 to the drain pipe 41 as the dilution liquid D to the mixing tank 32. The digested liquid pump 64 is configured to adjust the amount of the digested liquid I to be supplied to the mixing tank 32 such that the concentration of the coolant mixture M reaches the predetermined target concentration based on the concentration of the coolant mixture M measured by the second concentration measuring unit 61 and the concentration of the digested liquid I measured by the third concentration measuring unit 63.

The third concentration measuring unit 63 of the present embodiment is provided between the drain pipe 41 and the digested liquid pump 64, and is configured to measure the concentration of the digested liquid I flowing from the drain pipe 41 to the digested liquid pump 64. The third concentration measuring unit 63 can take various forms as long as it is configured to measure the concentration of the digested liquid I. For example, the third concentration measuring unit 63 may be a concentration meter configured to measure the concentration of the digested liquid I based on various physical property values such as a refractive index, density, and conductivity of the digested liquid I.

The third concentration measuring unit 63 preferably includes a COD sensor configured to measure the concentration based on the chemical oxygen demand of the digested liquid I. In this case, the third concentration measuring unit 63 can measure the concentration of the digested liquid I based on the COD of the digested liquid I. The dilution device 6 can prepare a coolant mixture M that is more easily fermentable by setting the supply amount of the dilution liquid D based on the determined concentration of the digested liquid I. As a result, the efficiency of production of the biogas G can further be increased.

The biogas production system 103 of the present embodiment is configured such that at least part of the digested liquid I can be used as the dilution liquid D. By reusing at least part of the digested liquid I as the dilution liquid D and circulating it through the biogas production system 103, the total amount of the digested liquid I discharged from the biogas production system 103 can further be reduced. Thus, the biogas production system 103 can be made compact more easily. In this case, the amount of the tap water W used as the dilution liquid D can be reduced. Thus, the environmental load caused by the operation of the biogas production system 103 can further be reduced.

The biogas production system 103 is also configured such that both the tap water W and the digested liquid I can be used as the dilution liquid D. Therefore, for example, when the efficiency of fermentation of the coolant mixture M is not sufficiently high immediately after the operation of the biogas production system 103, the concentration of the coolant mixture M can be adjusted using the tap water W, and the efficiency of fermentation of the coolant mixture M can be increased quickly. When the biogas production system 103 is in steady operation and the efficiency of fermentation of the coolant mixture M is sufficiently high, the concentration of the coolant mixture M can be adjusted using the digested liquid I.

### (Fourth Embodiment)

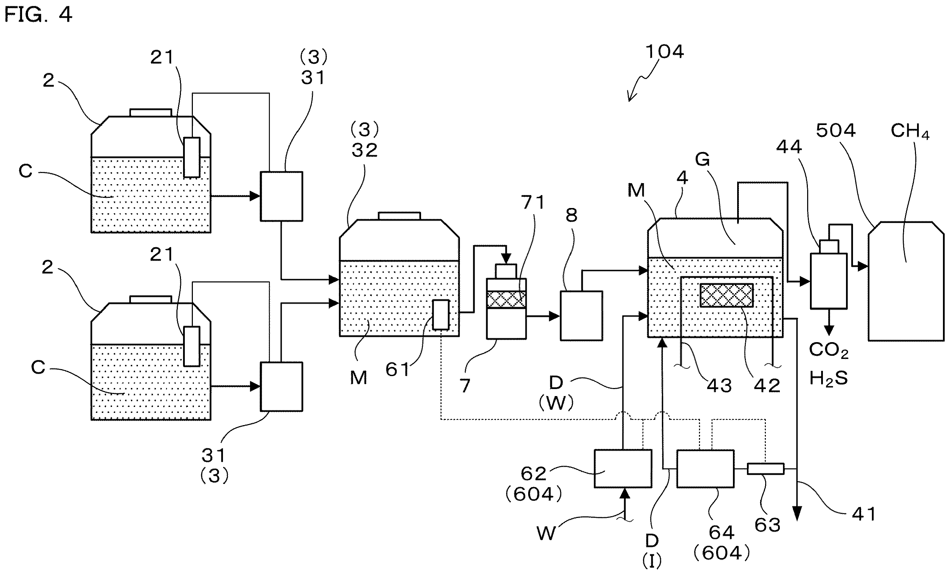

The present embodiment illustrates an example of a biogas production system 104 including a metal removal device 7 and an inhibitor conversion device 8. As shown in FIG. 4, the biogas production system 104 of the present embodiment includes the plurality of storage tanks 2, the concentration measuring units 21 configured to measure the concentrations of the waste water-soluble coolants C in the storage tanks 2, the mixing device 3 configured to mix the waste water-soluble coolants C in the storage tanks 2 to prepare the coolant mixture M, and the fermentation device 4 configured to ferment the coolant mixture M. The configurations of the storage tank 2, the concentration measuring unit 21, the mixing device 3, and the fermentation device 4 in the biogas production system 104 are similar to those in the biogas production system 1 of the first embodiment. The mixing tank 32 of the mixing device 3 is provided with the second concentration measuring unit 61 configured to measure the concentration of the coolant mixture M. The configuration of the second concentration measuring unit 61 is similar to that in the biogas production system 102 of the second embodiment.

The biogas production system 104 further includes the third concentration measuring unit 63 configured to measure the concentration of the digested liquid I, and a dilution device 604 configured such that the tap water W and the digested liquid I can be used as the dilution liquid D. The configuration of the third concentration measuring unit 63 is similar to that in the biogas production system 103 of the third embodiment. The configuration of the dilution device 604 is similar to that of the dilution device 603 in the biogas production system 103 of the third embodiment, except that it is configured to supply the tap water W and the digested liquid I as the dilution liquid D to the fermentation device 4 and dilute the coolant mixture M in the fermentation device 4.

The biogas production system 104 of the present embodiment further includes the metal removal device 7, the inhibitor conversion device 8, a gas separation device 44, and a methane tank 504.

The metal removal device 7 is disposed upstream of the fermentation device 4, and is configured to remove metal components from the waste water-soluble coolant C and/or the coolant mixture M. The waste water-soluble coolant C and the coolant mixture M may contain, for example, metal powder and cutting chips generated during machining as metal components. These metal components may adversely affect the fermentation of the coolant mixture M in the fermentation device 4. As in the biogas production system 104 of the present embodiment, the metal removal device 7 is provided upstream of the fermentation device 4, and removes the metal components in the waste water-soluble coolant C and/or the coolant mixture M. Thus, the effect of the metal components on the fermentation of the coolant mixture M can be reduced. As a result, it is expected that the coolant mixture M in the fermentation device 4 can be fermented more efficiently.

Specifically, the metal removal device 7 may be disposed on the path of the waste water-soluble coolant C and the coolant mixture M from the storage tank 2 to the fermentation device 4. For example, the metal removal device 7 of the present embodiment is disposed between the mixing tank 32 and the fermentation device 4, and is configured to remove the metal components contained in the coolant mixture M.

The method for removing the metal components by the metal removal device 7 is not particularly limited, and any appropriate method can be adopted depending on the form and properties of the metal components contained in the waste water-soluble coolant C and the coolant mixture M. For example, the metal removal device 7 may include a filter 71 that filters out the metal components from the waste water-soluble coolant C and the coolant mixture M. In this case, the metal removal device 7 may include a single filter 71, or may include a plurality of types of filter 71 having different filtering capabilities. As the filter 71, for example, a known filter such as a mesh filter or a ceramic filter can be used.

Although illustration is omitted in the figure, the metal removal device 7 may include a device that separates the metal components by utilizing gravity, such as a settling tank that settles the metal components, a device that separates the metal components by utilizing centrifugal force, such as a liquid cyclone, or a device that separates the metal components by utilizing electromagnetic force, such as a magnetic separator. These devices may be used alone, or two or more types of device may be used in combination.

The inhibitor conversion device 8 is disposed upstream of the fermentation device 4, and is configured to convert fermentation inhibitors in the waste water-soluble coolant C and/or the coolant mixture M into other substances. The waste water-soluble coolant C and the coolant mixture M may contain additives such as surfactants and preservatives. These additives may act as fermentation inhibitors that adversely affect the fermentation of the coolant mixture M in the fermentation device 4. As in the biogas production system 104 of the present embodiment, the inhibitor conversion device 8 is provided upstream of the fermentation device 4, and converts the fermentation inhibitors in the waste water-soluble coolant C and/or the coolant mixture M into other substances. Thus, the effect of the fermentation inhibitors on the fermentation of the coolant mixture M can be reduced. As a result, it is expected that the coolant mixture M in the fermentation device 4 can be fermented more efficiently.

Specifically, the inhibitor conversion device 8 may be disposed on the path of the waste water-soluble coolant C and the coolant mixture M from the storage tank 2 to the fermentation device 4. For example, the inhibitor conversion device 8 of the present embodiment is disposed between the metal removal device 7 and the fermentation device 4, and is configured to convert the fermentation inhibitors contained in the coolant mixture M into other substances.

The method for converting the fermentation inhibitors into other substances by the inhibitor conversion device 8 is not particularly limited, and any appropriate method can be adopted depending on the type of the fermentation inhibitors contained in the waste water-soluble coolant C and the coolant mixture M. For example, the inhibitor conversion device 8 may be configured to convert the fermentation inhibitors into other substances by an electrical method such as electrolysis. The inhibitor conversion device 8 may be configured to convert the fermentation inhibitors into other substances using a chemical method such as adding an inhibitor treatment agent that reacts with the fermentation inhibitors. Further, the inhibitor conversion device 8 may be configured to convert the fermentation inhibitors into other substances using a physical method such as ultrasonic irradiation. The inhibitor conversion device 8 may be configured to implement one of these methods, or may be configured to implement a combination of two or more of these methods.

When the biogas production system 104 includes the metal removal device 7 and/or the inhibitor conversion device 8 as in the present embodiment, the dilution device 603 is preferably configured to supply the dilution liquid D downstream of the metal removal device 7 and the inhibitor conversion device 8. In this case, the concentration of the coolant mixture M can be adjusted to a desired concentration while avoiding an increase in the amount of liquid to be treated in the metal removal device 7 and the inhibitor conversion device 8. Thus, the biogas production system 104 can be made compact more easily.

The gas separation device 44 is connected to the fermentation device 4, and is configured to separate methane and gases other than methane in the biogas G generated in the fermentation device 4. The methane separated in the gas separation device 44 is stored in the methane tank 504.

The biogas production system 104 including the gas separation device 44 can efficiently produce methane that is a highly useful resource. The methane produced in this manner can be used for various purposes, such as fuel for power generation, a gas material for carburizing heat treatment, and a raw material for chemical compounds.

Although illustration is omitted in the figure, the gas separation device 44 may be configured to separate carbon dioxide from the biogas G. In this case, carbon dioxide can also be reused as a resource in addition to methane.

The specific forms of the biogas production system according to the present disclosure have been described above based on the first to fourth embodiments. However, the specific forms of the biogas production system according to the present disclosure are not limited to the forms shown in the above embodiments, and the configuration can be changed as appropriate without departing from the spirit of the present disclosure.

For example, the second embodiment illustrates the example of the biogas production system 102 configured to dilute the coolant mixture M using the tap water W as the dilution liquid D. However, a configuration that uses the digested liquid I as the dilution liquid D can be adopted instead of the configuration that uses the tap water W as the dilution liquid D. In this case, for example, the drain pipe 41 and the mixing tank 32 may be connected by the digested liquid pump, the third concentration measuring unit may be provided between the drain pipe 41 and the digested liquid pump, and the supply amount of the dilution liquid D may be adjusted based on the concentration of the coolant mixture M measured by the second concentration measuring unit and the concentration of the digested liquid I measured by the third concentration measuring unit.

For example, the fourth embodiment illustrates the example of the biogas production system 104 configured such that both the tap water W and the digested liquid I can be used as the dilution liquid D. However, only either of the tap water W and the digested liquid I can be used as the dilution liquid D. For example, when only the tap water W is used as the dilution liquid D, the third concentration measuring unit 63 and the digested liquid pump 64 may be removed from the biogas production system 104. For example, when only the digested liquid I is used as the dilution liquid D, the water pump 62 may be removed from the biogas production system 104.

## Claims

1. A biogas production system configured to produce biogas using, as raw materials, waste water-soluble coolants collected from machining devices that perform machining, the biogas production system comprising:
a plurality of storage tanks configured to separate by type and store the waste water-soluble coolants collected from the machining devices;
concentration measuring units configured to measure concentrations of the waste water-soluble coolants in the storage tanks;
a mixing device configured to prepare a coolant mixture containing a plurality of the waste water-soluble coolants by mixing the waste water-soluble coolants in the storage tanks at a ratio calculated based on the concentrations of the waste water-soluble coolants; and
a fermentation device configured to generate the biogas by fermenting the coolant mixture supplied from the mixing device using microorganisms.

2. The biogas production system according to claim 1, wherein each of the concentration measuring units includes a COD sensor configured to measure a chemical oxygen demand of the waste water-soluble coolant, and is configured to measure the concentration based on the chemical oxygen demand of the waste water-soluble coolant.

3. The biogas production system according to claim 1 or 2, further comprising:
a dilution device configured to supply, to at least one type of liquid selected from the group consisting of the waste water-soluble coolant and the coolant mixture, a dilution liquid for diluting the liquid; and
a second concentration measuring unit configured to measure a concentration of the coolant mixture, wherein the dilution device is configured to adjust a supply amount of the dilution liquid based on the concentration of the coolant mixture.

4. The biogas production system according to claim 3, wherein the second concentration measuring unit includes a COD sensor configured to measure a chemical oxygen demand of the coolant mixture, and is configured to measure the concentration based on the chemical oxygen demand of the coolant mixture.

5. The biogas production system according to claim 3, further comprising a third concentration measuring unit configured to measure a concentration of a digested liquid made from the coolant mixture fermented in the fermentation device, wherein the dilution device is configured to use the digested liquid as the dilution liquid, and adjust the supply amount of the dilution liquid based on the concentration of the coolant mixture and the concentration of the digested liquid.

6. The biogas production system according to claim 5, wherein the third concentration measuring unit includes a COD sensor configured to measure a chemical oxygen demand of the digested liquid, and is configured to measure the concentration based on the chemical oxygen demand of the digested liquid.

7. The biogas production system according to claim 3, further comprising a metal removal device disposed upstream of the fermentation device, and configured to remove a metal component in the waste water-soluble coolant and/or the coolant mixture.

8. The biogas production system according to claim 3, further comprising an inhibitor conversion device disposed upstream of the fermentation device, and configured to convert a fermentation inhibitor in the waste water-soluble coolant and/or the coolant mixture into another substance.

9. The biogas production system according to claim 8, wherein the dilution device is configured to supply the dilution liquid downstream of the inhibitor conversion device.

10. The biogas production system according to claim 1 or 2, wherein the fermentation device is configured to generate the biogas containing methane.

11. The biogas production system according to claim 10, further comprising a gas separation device configured to separate the methane from the biogas generated in the fermentation device.

12. The biogas production system according to claim 11, further comprising a methane tank configured to store the methane separated in the gas separation device.

13. The biogas production system according to claim 1 or 2, wherein the machining is cutting or grinding.

## Amended claims

### Amended claims under Art. 19.1 PCT

**1.** A biogas production system configured to produce biogas using, as raw materials, waste water-soluble coolants collected from machining devices that perform machining, the biogas production system comprising:
a plurality of storage tanks configured to separate by type and store the waste water-soluble coolants collected from the machining devices;
concentration measuring units configured to measure concentrations of the waste water-soluble coolants in the storage tanks;
a mixing device configured to prepare a coolant mixture containing a plurality of the waste water-soluble coolants by mixing the waste water-soluble coolants in the storage tanks at a ratio calculated based on the concentrations of the waste water-soluble coolants;
a fermentation device configured to generate the biogas by fermenting the coolant mixture supplied from the mixing device using microorganisms;
a dilution device configured to supply, to at least one type of liquid selected from the group consisting of the waste water-soluble coolant and the coolant mixture, a dilution liquid for diluting the liquid; and
a second concentration measuring unit configured to measure a concentration of the coolant mixture, wherein
the dilution device is configured to adjust a supply amount of the dilution liquid based on the concentration of the coolant mixture.

**2.** The biogas production system according to claim 1, wherein each of the concentration measuring units includes a COD sensor configured to measure a chemical oxygen demand of the waste water-soluble coolant, and is configured to measure the concentration based on the chemical oxygen demand of the waste water-soluble coolant.

**3.** The biogas production system according to claim 1 or 2, wherein the second concentration measuring unit includes a COD sensor configured to measure a chemical oxygen demand of the coolant mixture, and is configured to measure the concentration based on the chemical oxygen demand of the coolant mixture.

**4.** The biogas production system according to claim 1 or 2, further comprising a third concentration measuring unit configured to measure a concentration of a digested liquid made from the coolant mixture fermented in the fermentation device, wherein the dilution device is configured to use the digested liquid as the dilution liquid, and adjust the supply amount of the dilution liquid based on the concentration of the coolant mixture and the concentration of the digested liquid.

**5.** The biogas production system according to claim 4, wherein the third concentration measuring unit includes a COD sensor configured to measure a chemical oxygen demand of the digested liquid, and is configured to measure the concentration based on the chemical oxygen demand of the digested liquid.

**6.** The biogas production system according to claim 1 or 2, further comprising a metal removal device disposed upstream of the fermentation device, and configured to remove a metal component in the waste water-soluble coolant and/or the coolant mixture.

**7.** The biogas production system according to claim 1 or 2, further comprising an inhibitor conversion device disposed upstream of the fermentation device, and configured to convert a fermentation inhibitor in the waste water-soluble coolant and/or the coolant mixture into another substance.

**8.** The biogas production system according to claim 7, wherein the dilution device is configured to supply the dilution liquid downstream of the inhibitor conversion device.

**9.** The biogas production system according to any one of claims 1 to 8, wherein the fermentation device is configured to generate the biogas containing methane.

**10.** The biogas production system according to claim 9, further comprising a gas separation device configured to separate the methane from the biogas generated in the fermentation device.

**11.** The biogas production system according to claim 10, further comprising a methane tank configured to store the methane separated in the gas separation device.

**12.** The biogas production system according to any one of claims 1 to 11, wherein the machining is cutting or grinding.

**13.**
